# FASCICULE DE BREVET EUROPEEN

(11) **EP 1 078 266 B1**
(45) Date de publication et mention de la délivrance du brevet: **17.11.2004**
(21) Numéro de dépôt: 99919325.3
(22) Date de dépôt: 12.05.1999
(51) Int. Cl.: G01N 33/68, G01N 33/74, G01N 33/574, G01N 33/577, C07K 16/26

(54) **DOSAGE IMMUNOLOGIQUE DE LA CHROMOGRANINE A HUMAINE (CgA), ANTICORPS, REACTIFS ET TROUSSES UTILISABLES POUR CE DOSAGE**
IMMUNTEST FÜR HUMAN CHROMOGRANINE A (CgA), ANTIKÖRPER, REAGENZ UND DAZU GEEIGNETER TESTSATZ
HUMAN CHROMOGRANIN A (GgA) IMMUNOLOGIC ASSAY, ANTIBODIES, REAGENTS AND KITS FOR SAID ASSAY

(30) Priorité: 14.05.1998 FR 9806101
(43) Date de publication de la demande: 28.02.2001
(73) Titulaire: CIS BIO INTERNATIONAL, 91400 Saclay (FR)
(72) Inventeur: DEGORCE, François, F-30430 Saint Privat de Champclos (FR); BELLANGER, Laurent, F-84220 Cabrières d'Avignon (FR); AUNIS, Dominique, F-67100 Strasbourg (FR)
(74) Mandataire: Audier, Philippe André
(86) Numéro de dépôt international: PCT/FR1999/001141
(87) Numéro de publication internationale: WO 1999/058980

(56) Documents cités:
- EP-A- 0 449 269
- US-A- 4 758 522
- DEGORCE, FRANCOIS (1) ET AL: "Selection of monoclonal antibodies for the measurement of chromogranin A by sandwich assay." TUMOR BIOLOGY, (1997) VOL. 18, NO. SUPPL. 1, PP. 112. MEETING INFO.: 24TH MEETING OF THE INTERNATIONAL SOCIETY FOR ONCODEVELOPMENTAL BIOLOGY AND MEDICINE ON THE INTERDEPENDENCE OF TUMOR BIOLOGY AND CLINICAL ONCOLOGY SAN DIEGO, CALIFORNIA, USA NOVEMBE, XP002094255

## Description

### Domaine technique

La présente invention a pour objet un procédé de dosage immunologique de la chromogranine A humaine, qui permet en particulier de doser non seulement la chromogranine A sous forme intacte mais également les fragments majeurs de cette chromogranine A.

Un tel dosage peut être utilisé en particulier pour le diagnostic et le suivi de pathologies telles que par exemple le phéochromocytome et les carcinoïdes intestinaux.

### Etat de la technique antérieure

La chromogranine A (CgA) est une protéine d'un poids moléculaire de 48 kDa, d'un pI de 4,9, dont la forme humaine comporte 439 acides aminés comme il est décrit par Konecki et al, 1987, dans la référence [1]. Elle appartient à la famille des granines avec lesquelles elle partage des similitudes structurales et physiologiques. La CgA présente tout comme la chromogranine B une conservation interespèce marquée qui suppose une fonction majeure. La CgA est largement représentée dans les sécrétagogues des cellules endocrines et neuroendocrines dont elle constitue avec les autres granines une des composantes principales. Elle intervient à ce niveau à la fois comme élément régulateur de la cosecrétion d'autres entités, comme les catécholamines dans la glande surrénale.

La CgA joue en outre un rôle essentiel de prohormone par le biais de la libération de peptides actifs, consécutive à la dégradation protéolytique intra-granulaire et extra-matricielle.

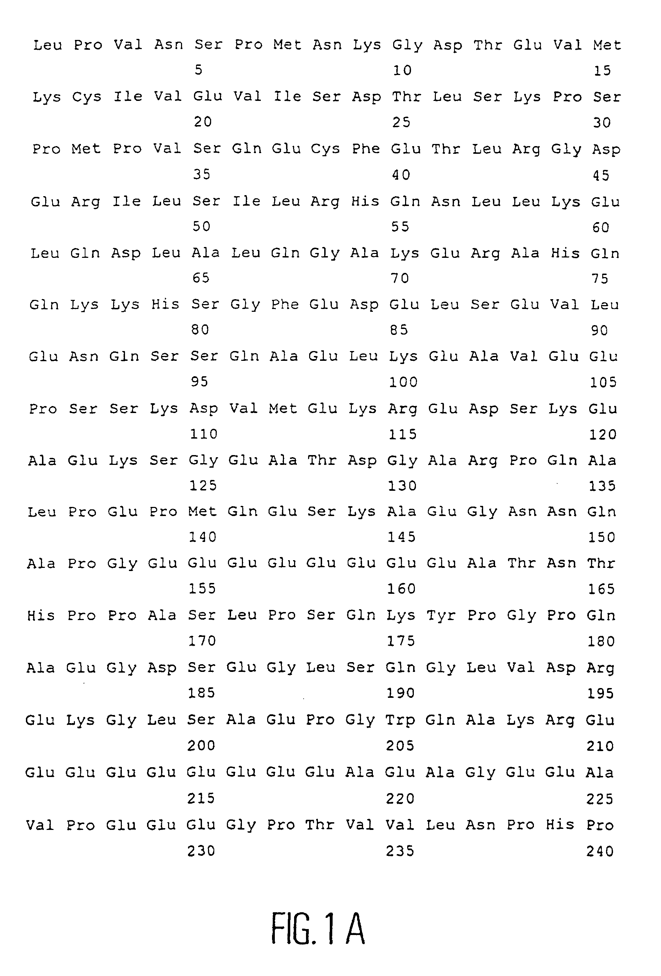

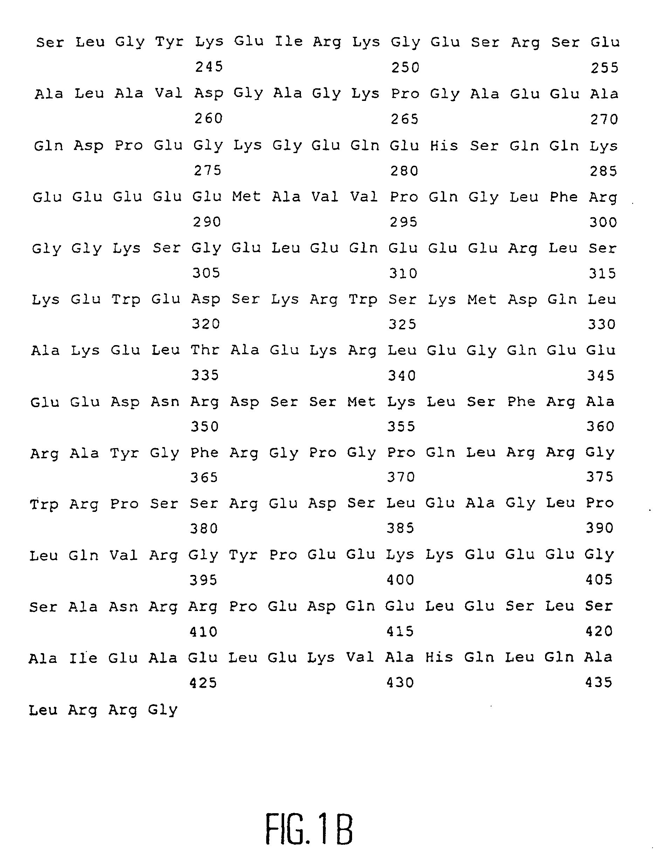

Sur la figure 1 annexée, on a représenté la séquence n° 1 des 439 acides aminés décrite par Konecki et al, correspondant à la chromogranine A humaine.

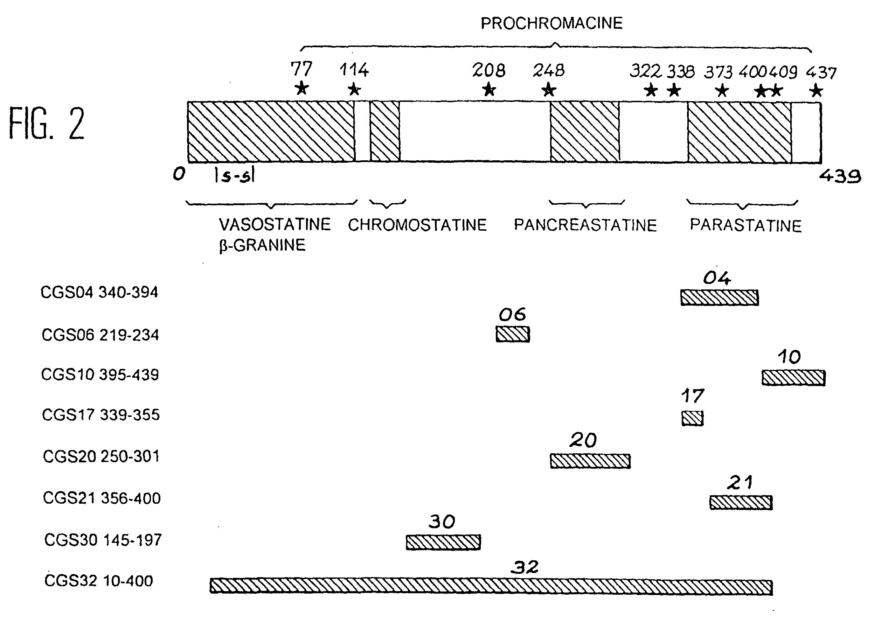

Sur la figure 2, on a représenté cette séquence de façon simplifiée, en identifiant sur cette figure certains des peptides susceptibles d'être libérés chez l'homme, soit les peptides : vasostatine/β granine, chromostatine, pancréastatine, parastatine et prochromacine. La protéolyse touche les sites dibasiques numérotés et repérés par un astérisque sur la figure 2, qui sont répartis le long de la séquence de la CgA et sont au nombre de 10 dans la CgA humaine. Cette protéolyse a été décrite comme un phénomène récurrent de part et d'autre des extrémités de la protéine et il a été démontré qu'elle était spécifique des tissus et qu'elle conduisait à des différences importantes dans la distribution tissulaire des peptides produits. Le degré d'intensité et la nature de la protéolyse seraient donc responsables de la grande variabilité des fragments retrouvés dans les tissus, la circulation sanguine et l'urine.

Les travaux récents de Corti et al dans la référence [2] ont confirmé cette diversité en montrant qu'il existe chez les patients atteints de phéochromocytome des formes circulantes de conformation différente et en proportions variables d'un individu à l'autre. De la même manière, il a été montré, à travers l'étude du peptide WE-14, que la CgA était protéolysée de façon différente dans les tissus normaux et les tissus néoplasiques du tractus gastro-entero-pancréatique.

Outre sa mise en évidence dans les tissus normaux ainsi que dans les néoplasies correspondantes, de nombreuses études attribuent au dosage de la CgA un intérêt diagnostique. Les taux de CgA circulante sont significativement élevés dans les cas de phéochromocytome, de carcinoïde et de tumeur endocrine du pancréas. Ces données ont été confirmées puis élargies à d'autres pathologies : neuroblastome, tumeurs du tractus gastro-intestinal, hypertension essentielle. La présence de CgA a été démontrée dans un nombre important de pathologies neurodégénératives dont l'Alzheimer (voir la référence Munoz, Lab. Invest., 1991, vol. 64, pages 826-832 [15]). Certains auteurs ont également montré que la présence de CgA dans les cancers prostatiques pouvait être le signe d'une évolution défavorable, de même que dans les cas de cancer du rein. Le dosage de la CgA présente donc un grand intérêt.

Degorce et al dans la référence [16] indiquent, sur la base d'une étude visant à sélectionner des combinaisons d'anticorps monoclonaux pour mesurer la CgA humaine par un dosage immunologique, que celle-ci renferme trois groupes principaux d'épitopes : un groupe médian allant de l'acide aminé n° 136 à l'acide aminé n° 340, un groupe intermédiaire allant de l'acide aminé n° 340 à l'acide aminé n° 394 et un groupe C-terminal allant de l'acide aminé n° 394 à l'acide aminé n° 439.

Le document US-A-4 758 522 [3] décrit un dosage immunologique de la CgA dans lequel la CgA est mesurée par compétition avec de la CgA radiomarquée pour les sites d'un anticorps anti-CgA humaine.

Syversen et al dans la référence [4] ont décrit un dosage de la chromogranine A par la méthode ELISA utilisant un anticorps dirigé contre un fragment C-terminal de la CgA correspondant à la séquence des acides aminés n° 210 à 439, et un dosage radioimmunologique de la pancréastatine.

Corti et al dans la référence [2] ont également décrit deux dosages de la CgA utilisant des anticorps monoclonaux dirigés contre les séquences d'acides aminés 81 à 90 et 68 à 70 de la CgA humaine.

Ces dosages prennent donc en considération certains fragments de la CgA, mais ils ne permettent pas de différencier le type de pathologie à partir des résultats du dosage.

En effet, la protéolyse de la molécule et la multiplicité des fragments circulants supposent une configuration de dosage qui soit capable de détecter la majorité de ces entités.

La présente invention a précisément pour objet un procédé de dosage immunologique de la CgA qui mesure les taux de la CgA intacte ainsi que les taux des fragments majeurs retrouvés au niveau de la circulation sanguine.

### Exposé de l'invention

Selon l'invention, le procédé de dosage immunologique de la chromogranine A (CgA) humaine présente dans un échantillon comprend l'utilisation d'au moins un anticorps monoclonal ou polyclonal qui se lie spécifiquement à un épitope situé dans la séquence d'acides aminés n° 2 allant de l'acide aminé n° 145 à l'acide aminé n° 234 à partir de l'extrémité N-terminale de la CgA humaine.

Selon l'invention, le terme dosage immunologique recouvre tous les types de dosage immunologique, en phase homogène ou en phase hétérogène, tels que par exemple les dosages par compétition, les dosages par la méthode sandwich, les dosages ELISA, RIA, et tout autre type de dosage mettant en oeuvre une réaction antigène-anticorps.

Selon un premier mode de réalisation de l'invention, le dosage immunologique est un dosage par compétition entre la chromogranine A de l'échantillon et une quantité donnée de chromogranine A humaine (hCgA) marquée, pour les sites dudit anticorps.

Pour mettre en oeuvre ce dosage, on ajoute à l'échantillon à doser une quantité de CgA humaine marquée et l'anticorps qui se lie spécifiquement à un épitope situé dans la séquence n° 2 de la CgA, puis on laisse incuber. De la sorte, on a une compétition entre la CgA de l'échantillon et la CgA marquée pour les sites de cet anticorps. Lorsqu'on réalise le dosage en phase hétérogène, en utilisant comme marqueur un radioélément, on peut séparer les complexes anticorps-hCgA formés par immuno-précipitation au moyen d'un anticorps approprié. En déterminant ensuite la radioactivité des complexes formés, on peut déterminer la concentration en CgA de l'échantillon en se référant à une courbe standard obtenue à partir d'échantillons standards de concentrations connues en CgA.

On peut aussi réaliser le dosage en phase homogène en utilisant par exemple de la hCgA marquée et un anticorps apte à modifier le signal émis par la hCgA marquée.

Dans ce dosage, on utilise avantageusement l'anticorps spécifique d'un épitope situé dans la séquence d'acides aminés n° 3 allant de l'acide aminé n° 219 à l'acide aminé n° 234 de la CgA humaine, ou l'anticorps spécifique d'un épitope situé dans la séquence d'acides aminés n° 4 allant de l'acide aminé n° 145 à l'acide aminé n° 197 de la CgA humaine. Ces anticorps sont de préférence des anticorps monoclonaux.

Pour réaliser ce dosage, on peut utiliser pour la préparation des échantillons standards et de la CgA marquée, de la CgA humaine purifiée provenant de cellules endocrines ou neuroendocrines d'origine humaine ou, de préférence, de la CgA humaine recombinante.

Selon un second mode de réalisation de l'invention, le dosage immunologique est un dosage de type sandwich utilisant un premier anticorps monoclonal ou polyclonal qui se lie spécifiquement à un premier épitope de la CgA humaine, et un second anticorps monoclonal ou polyclonal qui se lie spécifiquement à un second épitope de la CgA humaine différent du premier, l'un au moins des épitopes étant situé dans la séquence n° 2 d'acides aminés de la CgA humaine et l'un des deux anticorps étant marqué.

Dans ce second mode de réalisation de l'invention, on utilise deux anticorps spécifiques d'épitopes différents, dont l'un au moins est situés dans la séquence n° 2 de la CgA. Ce dosage peut être effectué en phase hétérogène en immobilisant le premier anticorps sur une phase solide et en utilisant un second anticorps marqué.

Ce dosage peut également être effectué en phase homogène, en utilisant un premier anticorps marqué et un second anticorps apte à modifier le signal émis par le premier anticorps marqué.

Le premier et le second anticorps peuvent être choisis parmi les anticorps spécifiques d'épitopes situés dans les séquences d'acides aminés n° 145 à 197 (séquence 4) et/ou n° 219 à 234 (séquence 3) de la CgA humaine.

A titre d'exemple, le premier anticorps peut être spécifique d'un épitope situé dans la séquence d'acides aminés n° 145 à 197, (séquence n° 4) de la CgA et le second anticorps peut être spécifique d'un épitope situé dans la séquence d'acides aminés n° 219 à 234 (séquence n° 3) de la CgA.

On peut aussi utiliser dans ce dosage un premier anticorps spécifique d'un épitope situé dans la séquence d'acides aminés n° 219 à 234 (séquence n° 3) et un second anticorps spécifique d'un épitope situé dans la séquence d'acides aminés n° 145 à 197 (séquence n° 4) de la CgA humaine.

On peut également utiliser un premier anticorps spécifique d'un épitope situé dans la séquence d'acides aminés n° 145 à 197 (séquence n° 4) ou dans la séquence d'acides aminés n° 219 à 234 (séquence n° 3) de la CgA humaine, et un second anticorps spécifique d'un épitope situé dans la séquence d'acides aminés n° 250 à 301 (séquence n° 5) de la CgA humaine.

Selon un troisième mode de réalisation de l'invention, le dosage immunologique est un dosage de type sandwich utilisant un premier anticorps monoclonal ou polyclonal qui se lie spécifiquement à un épitope situé dans la séquence n° 2 de la CgA humaine et un second anticorps monoclonal ou polyclonal qui se lie spécifiquement à un second épitope situé dans la séquence n° 5 de la CgA humaine allant de l'acide aminé n° 250 à l'acide aminé n° 301, l'un des deux anticorps étant marqué.

Dans ce mode de réalisation de l'invention, le dosage peut être effectué en phase hétérogène en utilisant un premier anticorps immobilisé sur une phase solide et un second anticorps marqué, ou l'inverse. On peut aussi effectuer le dosage en phase homogène, en utilisant un premier anticorps marqué et un second anticorps apte à modifier le signal émis par le premier anticorps marqué, ou l'inverse.

Comme premier anticorps, on utilise avantageusement un anticorps spécifique de la séquence d'acides aminés n° 145 à 197 (séquence n° 4) ou un anticorps spécifique de la séquence d'acides aminés n° 219 à 234 (séquence n° 3) de la CgA humaine.

Le procédé de l'invention est particulièrement intéressant pour le diagnostic et le suivi de pathologies telles que le phéochromocytome ou les tumeurs carcinoïdes, car ils permettent de discriminer positivement les sérums de patients atteints de phéochromocytome ou de tumeurs carcinoïdes par rapport aux sérums normaux.

Pour mettre en oeuvre le procédé de l'invention, on peut utiliser des méthodes classiques de dosage immunologique en phase hétérogène, qui permettent de séparer les complexes CgA-anticorps de l'anticorps marqué en excès.

On peut aussi utiliser dans l'invention des méthodes de dosage en phase homogène qui ne comportent pas une telle séparation.

Les méthodes de dosage en phase homogène sont basées sur l'emploi d'un couple anticorps-antigène ou d'un couple de deux anticorps qui lorsqu'ils sont proches l'un de l'autre émettent un signal différent de celui qu'ils émettent séparément.

Dans ce but, on peut utiliser en particulier un anticorps couplé à un composé luminescent accepteur d'énergie et un anticorps couplé à un composé luminescent donneur d'énergie.

A titre d'exemple le composé luminescent accepteur d'énergie peut être l'allophycocyanine et le composé luminescent donneur d'énergie peut être un cryptate d'europium tel que ceux décrits dans EP-A-0 321 353 [11].

Une méthode de dosage en phase homogène particulièrement intéressante est la méthode TRACE® décrite dans la référence [14].

Dans le cas des dosages en phase hétérogène, on utilise une phase solide sur laquelle est immobilisé l'anticorps non marqué utilisé pour le dosage.

Les phases solides susceptibles d'être utilisées peuvent être de différents types. Ainsi, on peut utiliser des phases solides macroscopiques, constituées par des tubes, des billes ou des ailettes réalisés en polymère ou en d'autres matériaux.

Les polymères susceptibles d'être utilisés sont par exemple le polystyrène, les polyamides, le polypropylène, les polyoxyméthylènes et les copolymères de styrène.

On peut aussi utiliser des phases solides microscopiques finement divisées, par exemples des poudres et des agrégats en polymère, en protéine ou en d'autres matériaux.

Dans l'invention, le terme « marqué », appliqué à la CgA humaine dans le premier mode de réalisation de l'invention ou aux différents anticorps dans le second mode de réalisation, signifie que la CgA ou les anticorps ont été modifiés par un élément marqueur qui peut être, par exemple, un radioélément, un élément fluorescent, un élément luminescent, une enzyme, un chromophore fluorescent, un chromophore absorbant la lumière, ou tout autre ligand permettant une mesure quantitative directe ou indirecte.

L'invention concerne également les anticorps monoclonaux spécifiques d'un épitope situé soit dans la séquence n° 4 des acides aminés n° 145 à 197, soit dans la séquence n° 3 des acides aminés n° 219 à 234, de la chromogranine A humaine, et des réactifs immunologiques comportant l'un des anticorps lié à un marqueur détectable ou fixé sur une phase solide.

Ces anticorps sont particulièrement intéressant car ils conviennent à tous les types de dosage. En effet, ils donnent de bons résultats non seulement dans des dosages réalisés avec des marqueurs classiques (radioélément, etc...) mais aussi dans des dosages réalisés avec des marqueurs fluorescents du type cryptate et allophycocyanine qui généralement ne peuvent être utilisés qu'avec certains anticorps.

L'invention concerne de plus des trousses de dosage immunologique de la CgA humaine comportant les anticorps monoclonaux précités.

Elle concerne encore une trousse de dosage immunologique de la CgA humaine, qui comprend :
- un premier anticorps, et
- un second anticorps,
l'un des deux anticorps au moins étant un anticorps spécifique d'un épitope situé dans la séquence n° 2 des acides aminés n° 145 à 234 à partir de l'extrémité N-terminale de la CgA humaine.

Selon un premier mode de réalisation de la trousse, le premier anticorps et le second anticorps sont choisis parmi les anticorps spécifiques d'épitopes situés dans les séquences d'acides aminés n° 145 à 197 (séquence n° 4) et/ou n° 219 à 234 (séquence n° 3) de la CgA humaine.

Selon un second mode de réalisation de la trousse, l'un des anticorps est spécifique d'un épitope situé dans la séquence d'acides aminés n° 145 à 197 (séquence n° 4), ou dans la séquence d'acides aminés n° 219 à 234 (séquence n° 3), et l'autre anticorps est spécifique d'un épitope situé dans la séquence d'acides aminés n° 250 à 301 (séquence n° 5) de la CgA humaine.

De préférence, lorsque la trousse est destinée à un dosage en phase hétérogène, l'un des anticorps est immobilisé sur une phase solide et l'autre anticorps est couplé à un marqueur détectable.

Le marqueur détectable est par exemple l'iode 125.

De préférence, lorsque la trousse est destinée à un dosage en phase homogène, l'un des anticorps est couplé à un composé luminescent donneur d'énergie et l'autre anticorps est couplé à un composé luminescent accepteur d'énergie.

Avantageusement, le composé luminescent donneur d'énergie est un cryptate d'europium et le composé luminescent accepteur d'énergie est l'allophycocyanine.

Elle concerne de plus une trousse de dosage immunologique par compétition comprenant un anticorps spécifique d'un épitope situé dans la séquence n° 2 des acides aminés n° 145 à 234 de la CgA.

Dans cette trousse, l'anticorps est de préférence spécifique d'un épitope situé dans la séquence n° 3 des acides aminés n° 219 à 234 ou dans la séquence n° 4 des acides aminés n° 145 à 197 de la CgA humaine.

Selon l'invention, les anticorps utilisés pour le dosage peuvent être des anticorps polyclonaux ou des anticorps monoclonaux.

Les anticorps polyclonaux peuvent être obtenus à partir des fragments peptidiques de la chromogranine A humaine obtenus par protéolyse de la CgA humaine ou par synthèse peptidique, par immunisation in vivo ou in vitro. Dans ce cas, il est nécessaire de coupler le fragment peptidique à une protéine de transport telle que l'hémocyanine de la patelle (KLH), pour obtenir des anticorps chez l'animal.

Les anticorps monoclonaux peuvent être obtenus à partir d'hybridomes selon la technique de H. Kohler et C. Milstein décrite dans Nature, 1975, 256, pages 495-497. Dans ce cas, on réalise une ou plusieurs immunisations de souris avec la chromogranine A totale ou avec des fragments peptidiques correspondant aux épitopes voulus, associés à une protéine porteuse, puis on prélève la rate des animaux et on effectue une fusion cellulaire des splénocytes avec des cellules de myélome. On effectue ensuite un criblage des hybridomes produits pour sélectionner les clones produisant les anticorps spécifiques des épitopes de la CgA humaine.

De préférence, selon l'invention, on utilise des anticorps monoclonaux.

D'autres caractéristiques et avantages de l'invention apparaîtront mieux à la lecture de la description qui suit, d'exemples de réalisation donnés bien entendu à titre illustratif et non limitatif, en référence aux dessins annexés.

### Brève description des dessins

La figure 1 illustre la séquence n° 1 des 439 acides aminés de la CgA humaine mature.

La figure 2 illustre de façon simplifiée la séquence d'acides aminés de la CgA humaine avec les fragments obtenus par protéolyse et leur emplacement sur cette protéine. Les astérisques et les numéros qui leur sont associés indiquent les sites de clivage dibasiques. Les anticorps dirigés contre certains épitopes sont répertoriés en dessous.

La figure 3 représente le spectre obtenu lors de la chromatographie liquide à haute performance de CgA humaine recombinante après protéolyse par l'endoprotéase Lys-C.

La figure 4 est un diagramme représentant la distribution des différentes populations testées par un dosage par compétition (RIA) conforme à l'invention. Le nombre de cas testés est donné au-dessus de chaque groupe d'essai. Les lignes indiquent la médiane pour chaque groupe d'essai.

La figure 5 est un diagramme illustrant les résultats obtenus avec trois dosages de type sandwich par rapport au dosage par compétition en utilisant des anticorps différents. Les résultats sont exprimés sous la forme du rapport de concentration des plasmas pathologiques sur la valeur du 95è percentile d'une population normale (n=20). Les lignes indiquent la médiane pour chaque groupe d'essai.

La figue 6 représente la courbe obtenue, soit la concentration en CgA humaine d'un échantillon (en ng/ml) en fonction de la fluorescence détectée (F), en mettant en oeuvre un dosage en phase homogène conforme à l'invention.

La figure 7 est un diagramme illustrant la corrélation entre les résultats obtenus avec les dosages FIA et IRMA de la CgA (en ng/ml).

La figure 8 illustre les taux de CgA (en ng/ml) obtenus avec des échantillons normaux et des échantillons pathologiques en utilisant les dosages IRMA et FIA des exemples 2 et 3.

La description qui suit illustre la préparation d'anticorps monoclonaux dirigés contre des épitopes différents de la CgA humaine et la sélection des anticorps utiles pour le dosage immunologique de la chromogranine A humaine.

### I) - Production des anticorps monoclonaux

### 1 - Isolement et purification de la CgA humaine.

On utilise la méthode décrite par O'Connor et al dans la référence [6] pour purifier la hCgA à partir de glandes surrénales humaines de patients atteints de phéochromocytome. La séparation finale de la protéine est effectuée par électrophorèse sur gel 2-D en utilisant la technique originale de O'Farrel décrite dans la référence [7] et modifiée par Bader et Aunis comme il est décrit dans la référence [8]. La CgA est électroéluée à partir des gels. On produit également de la CgA humaine recombinante (rhCgA) à partir d'une souche de E. Coli BL21 (DE3) exprimant rhCgA comme il est décrit par Taupenot et al dans la référence [9].

### 2 - Marquage de la CgA purifiée (¹²⁵I-hCgA)

Les CgA purifiées native et recombinante sont marquées avec Na¹²⁵I selon la méthode à la chloramine T de Hunter et Greenwood à une activité spécifique de 1500 kBq µg⁻¹. L'iode libre est éliminé par filtration sur gel Sephadex-G25 (Pharmacia Biotech). Les fractions de protéine radiomarquée sont mélangées et diluées dans du PBS, pH 7,2 contenant 45 mmole/l de NaN₃, 0,5 % de sérum albumine bovine (BSA).

### 3 - Immunisations

Des souris Balb/c âgées de 6 à 8 semaines sont respectivement immunisées par voie intrapéritonéale avec 10 µg de hCgA ou de rhCgA purifiée obtenue précédemment dans de l'adjuvant complet de Freund. Les doses intrapéritonéales suivantes sont effectuées dans de l'adjuvant incomplet de Freund à des intervalles d'un mois. Les immunisations des souris sont suivies par titrage du sérum par immunoprécipitation avec ¹²⁵I-hCgA obtenue. précédemment.

### 4 - Production et criblage des hybridomes

On injecte ensuite par voie intraveineuse aux souris 10 µg de hCgA dans une solution de NaCl à 0,9 % trois jours avant la fusion cellulaire. On isole les cellules de la rate et on les fusionne avec des cellules de myélome P3-X63-Ag 8.653 en présence de polyéthylène glycol à 37 % (poids moléculaire 1540).

On identifie les clones positifs par immunoprécipitation avec ¹²⁵I-hCgA. Les anticorps provenant de clones positifs sont produits à partir d'ascites de souris et purifiés en utilisant le gel sépharose-protéine A.

Onze fusions ont été réalisées avec des souris immunisées contre la hCgA. Seulement deux d'entre elles ont abouti et généré 8 clones. Les deux fusions avec les animaux immunisés contre la protéine hCgA recombinante ont produit 16 clones de seconde génération. Au total 17 IgG1, 6 IgG2a et 1 IgG2b possédant des titres variant de 2 à 3236 ng/ml ont été produits pour être caractérisés.

### 5 - Identification des anticorps monoclonaux.

Les différentes familles d'épitopes de CgA ont été identifiées en testant les combinaisons possibles d'anticorps sur l'appareil Biacore® selon les instructions du fabricant. Tous les anticorps testés ont été dilués dans HEPES à 50 mmol/l, EDTA 15 mmol/l, NaCl 0,75 M, pH 7,4, et injectés à raison de 50 µg/ml. Le même tampon a été utilisé pour la CgA humaine recombinante, diluée à 20 µg/ml.

Cette analyse sur Biacore a permis de déterminer que les 24 anticorps testés se répartissent en huit groupes épitopiques comme il est indiqué dans le tableau 1. L'un d'entre eux (groupe 3) peut néanmoins être subdivisé en raison d'inhibitions partielles existant entre les anticorps de ce groupe.

La stoechiométrie de la réaction anticorps immobilisé -rhCgA varie de 0,157 à 0,621 mole d'antigène/mole d'anticorps (voir tableau 1).

Les anticorps de chaque groupe ou sous groupe reconnaissent à priori la même séquence.

### 6 - Identification des anticorps avec les peptides dérivés de la CgA recombinante humaine (rhCgA).

On fait digérer 2 nmol de rhCgA pendant 2 h à 37°C avec l'endoprotéinase Lys-C à un rapport protéine/protéinase de 1000 : 1 dans 100 mmole/l de TRIS-HCl, pH 8,3.

Les peptides dérivés de rhCgA produits sont alors séparés par HPLC sur une colonne de gel Macherey NAGEL 300-5C18 (250 mm x 4 mm). On mesure l'absorbance à 214 nm, et on utilise comme système solvant 0,1 % d'acide trifluoroacétique dans de l'eau (solvant A) et 0,1 % d'acide trifluoroacétique dans l'acétonitrile (solvant B). On élue les peptides à un débit de 0,7 ml/min en utilisant des gradients successifs du solvant B dans le solvant A de 0 à 25 % pendant 10 minutes et de 25 à 75 % pendant 50 minutes. Chaque fraction de pic est collectée et concentrée par évaporation mais pas jusqu'à siccité complète.

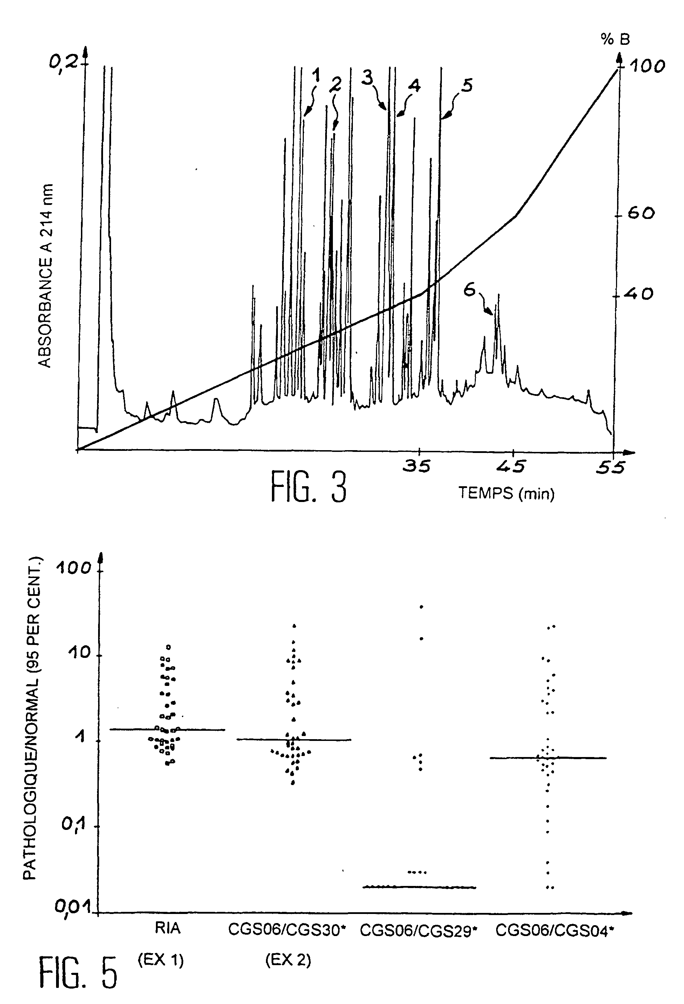

La figure 3 illustre les fractions recueillies en sortie de colonne en fonction du temps qui sont numérotées de 1 à 6.

La séquence des peptides purifiés est déterminée par dégradation automatique d'Edman sur un microséquenceur 473 A d'Applied Biosystems. Des échantillons purifiés par HPLC sont chargés sur des filtres de fibres de verre pré-cyclés et traités au polybrène. Les acides amino-phénylthiohydantoïne sont identifiés par chromatographie sur une colonne C-18 (PTH C-18, 2,1 mm x 200 mm). L'analyse par spectrométrie de masse est effectuée selon le processus décrit par Goumon et al dans la référence [10].

On utilise ces fractions pour identifier les anticorps monoclonaux obtenus précédemment. Dans ce but des parties aliquotes des fractions sont réparties sur des feuilles de nitrocellulose. Les membranes sont lavées rapidement avec NaCl/Pi (25 mM de phosphate de sodium pH 7,5 contenant 0,9 % de NaCl) et mises à incuber pendant 2 heures à la température ambiante avec les anticorps monoclonaux dilués au 1/1000 dans NaCl/Pi.

Le second anticorps est une IgG anti-souris conjuguée à la phosphatase alcaline. La réaction enzymatique prend place dans 100 mmol/l de TRIS-HCl, pH 8,5, 100 mmol/l de NaCl, 50 mmol/l de MgCl₂ contenant 0,4 mmol/l de nitro-bleu de tétrazolium et 0,38 mmol/l de 5-bromo-4-chloro-3-indolyl phosphate.

On teste un anticorps monoclonal de chacun des groupes du tableau 1, soit les anticorps CGS04, CGS06, CGS10, CGS30, CGS17, CGS21 et CGS32 produits précédemment, contre chaque fraction aliquote des fragments de rhCgA séparés par chromatographie liquide haute performance.

Le tableau 2 illustre la corrélation entre les différentes fractions et les anticorps reconnaissant ces fractions. Dans le tableau 2, on a donné également la masse des fragments isolés dans chaque fraction, leur position à partir de l'extrémité N-terminale de la chromogranine A ainsi que leur séquence N-terminale. Les anticorps CGS17 et CGS21 reconnaissent le domaine C-terminal de la protéine avec une haute spécificité pour les régions 339-355 et 356-400. Les anticorps CGS04 et CGS10 sont directement testés en Western-blot et les fragments immuno-détectés sont situés dans la région C-terminale de CgA (340-394 et 395-439).

Les anticorps CGS06, CGS20 et CGS30 sont spécifiques de la séquence n° 2 de la protéine et correspondent aux fragments 198-245, 246-303 et 145-197 (séquence n° 4), respectivement. Finalement, on remarque que l'anticorps CGS32 est fortement réactif avec un fragment important qui va de 10 à 400. Compte tenu du comportement de cet anticorps en association avec d'autres anticorps reconnaissant diverses régions de la CgA, il semble reconnaître plus spécifiquement la région 145-234 (séquence n° 2).

Parallèlement la séquence de l'épitope de l'anticorps CGS06 a été précisée par des peptides synthétiques. En effet, la séquence 198-245 montre une forte proportion d'acides glutamiques, région particulièrement hydrophile et potentiellement immunogène. Elle comporte notamment une première zone de 9 acides glutamiques contigus (210-218) et surtout une séquence de 3 acides glutamiques encadrée par deux prolines (227-232). Deux peptides, biotinylés en position N-terminale, correspondant aux séquences 207-234 et 219-245 ont été synthétisés. Ces peptides ont été immobilisés sur des tubes préalablement revêtus avec de la streptavidine. Les anticorps radiomarqués CGS30, CGS06, et CGS20 (2 kBq par ml) ont été incubés pendant 4 heures à température ambiante sous agitation avec chacun des peptides. Les tubes ont ensuite été lavés, puis comptés. Les deux anticorps CGS20 et CGS30 ne reconnaissent aucun des deux peptides. En revanche, CGS06 montre une fixation significative sur les deux séquences. La séquence minimale déduite correspond donc à la séquence n° 3, soit à la région 219-234, zone qui englobe le site poly-Glu 227-232 mentionné plus haut.

De la même manière, l'épitope de CGS20 a été précisé par rapport à la séquence de la pancréastatine. Dans ce cas, un peptide synthétique correspondant à la pancréastatine (séquence n° 5 des acides aminés 250-301 de la CgA humaine), a été rajouté en quantités croissantes dans la deuxième incubation des dosages immunoradiométriques suivants : CGS06/CGS30* et CGS06/CGS20*. Dans le premier cas aucune inhibition du signal n'est visible, prouvant ainsi que ni CGS06, ni CGS30 ne reconnaissent la séquence 250-301. Pour le système CGS06/CGS20* en revanche, une inhibition croissante est obtenue lorsque les quantités de peptide introduit augmentent (86 % d'inhibition pour 0.5 µg de peptide par tube). CGS20 reconnaît donc bien la séquence n°5 des acides aminés 250-301 de la CgA humaine, séquence correspondante à la pancréastatine.

Sur la figure 2, on a illustré la position de ces anticorps par rapport à la molécule totale de la chromogranine A en indiquant par ailleurs les séquences reconnues par ces anticorps.

Selon l'invention, on a trouvé que les anticorps CGS06, CGS20 et CGS30 (ou CGS33) permettaient de réaliser des dosages immunologiques spécifiques, reproductibles et précis. Les anticorps CGS30 (ou CGS33) et CGS06 peuvent être utilisés dans un dosage immunologique par compétition.

Les anticorps CGS06, CGS20 et CGS30 (ou CGS33) peuvent être couplés deux à deux pour réaliser un dosage de type sandwich.

Les exemples qui suivent illustrent des dosages de ce type.

### Exemple 1 : Dosage radioimmunologique de la chromogranine A humaine.

Dans cet exemple, on utilise pour le dosage l'anticorps CGS06 qui est spécifique de la séquence n° 3, soit la région allant de l'acide aminé 219 à l'acide aminé 234 de la chromogranine A. Pour effectuer ce dosage, on prépare des échantillons standards de chromogranine humaine recombinante (rhCgA) dilués dans du sérum humain normal. Les concentrations en rhCgA des échantillons standards vont de 0 jusqu'à 1 600 ng/ml.

On utilise par ailleurs de la rhCgA marquée à l'iode 125 comme il a été décrit précédemment.

Pour effectuer le dosage, on introduit dans un tube 50 µl de l'échantillon standard ou de l'échantillon à doser, 300 µl de hCgA radiomarquée à raison de 2 kBq par tube et 150 µl de la solution d'anticorps CGS06 purifiée à raison de 80 ng/ml. On réalise une première incubation pendant 24 heures à la température ambiante sous agitation légère. On sépare ensuite les complexes liés pendant 20 minutes à la température ambiante en ajoutant 75 µl d'immunoglobulines anti-souris de mouton diluées au 1/10è dans du PBS et 50 µl de sérum humain normal. On ajoute 1 ml de polyéthylène glycol à 6 % (poids moléculaire 6000) et on sépare les complexes précipités par centrifugation à 2000 g pendant 15 minutes. On détermine ensuite leur radioactivité dans un compteur gamma.

On obtient à partir des échantillons standards la courbe standard et on détermine la concentration en chromogranine A de l'échantillon à doser en repérant la valeur trouvée sur cette courbe standard.

La limite de détection déterminée par la répétabilité de la mesure au niveau du standard 0 (Bmax-2σ pour n=10 mesures) a été calculée à 14 ng/mL. Le dosage en 10 points de 2 sérums de concentration 243 et 450 ng/mL, a permis d'évaluer une précision intra-essai de 0.9 % sur les deux échantillons. Parallèlement, le test de précision inter-essai réalisé par le dosage de ces mêmes sérums dans 3 expériences différentes montre un CV de 4,5 et 6,4 % respectivement.

Ce dosage a été testé sur une série de 46 plasmas EDTA provenant de sujets sains, ainsi que sur 211 autres plasmas de patients atteints de pathologie neuroendocrine ou non.

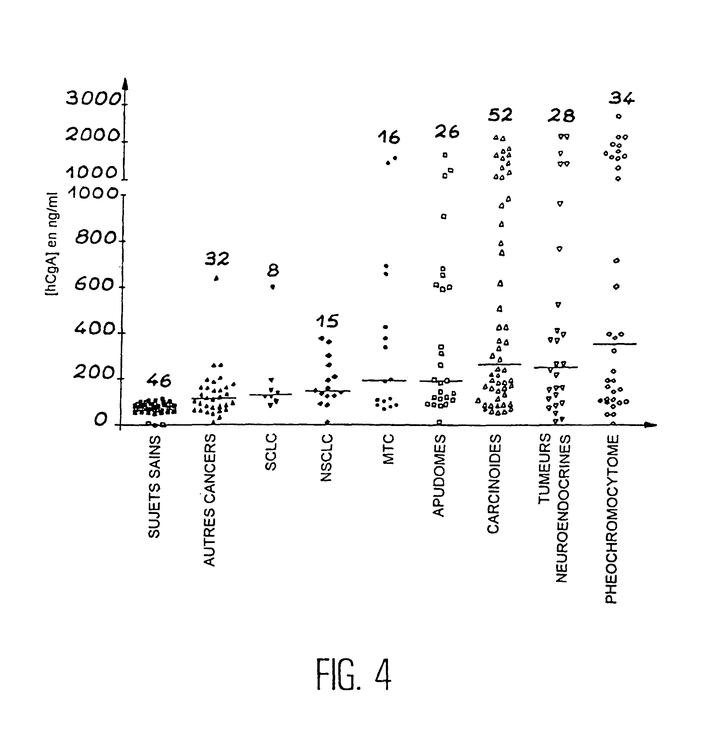

La figure 4 illustre les résultats obtenus avec cet essai sur les différents plasmas testés. Sur cette figure on a indiqué la nature des pathologies et le nombre de cas dans chaque groupe de patients.

L'analyse de variance en comparant deux à deux la population normale et chacune des autres populations montre que dans chaque cas, les taux de CgA sont significativement plus élevés dans les plasmas pathologiques (p<0,0001). En revanche, lorsque les différents groupes de plasmas sont comparés par rapport à la série de plasmas provenant de cancers non neuroendocrines, l'analyse montre que les groupes concernant les cancers du poumon à petites cellules (SCLC) ou non à petites cellules (NSCLC), ne sont pas significativement différents de la population de référence (p=0,2863 et 0,1798 respectivement). Les autres groupes étudiés présentent des valeurs en CgA significativement plus importantes particulièrement en ce qui concerne les carcinoïdes et les phéochromocytomes (p<0,0001) ainsi que les apudomes et autres tumeurs neuroendocrines (p<0,001).

Dans ce dosage, les taux de CgA trouvés ont été les suivants :
- 40 ng/ml pour le pool (NP) de plasmas des sujets sains.
- 675 nm/ml pour le pool (PP) de plasmas des sujets atteints de phéochromocytome et
- 1600 ng/ml pour le pool (CP) de plasmas des sujets atteints de tumeurs carcinoïdes intestinales.

### Exemple 2 : Dosage de la chromogranine A par une méthode sandwich (méthode IRMA).

Pour ce dosage, on utilise deux anticorps différents, le premier anticorps est l'anticorps CGS06 spécifique de la séquence n° 3 d'acides aminés 219-234 et le second anticorps est l'anticorps CGS30 spécifique de la séquence n° 4 d'acides aminés 145 à 197. Le premier anticorps est amené à une concentration de 10 µg/ml dans du PBS, pH 7,5, et on revêt des tubes de polystyrène avec cet anticorps.

Le second anticorps CGS30 est marqué à l'iode 125 au moyen de Na¹²⁵I en utilisant la méthode à la chloramine T, décrite précédemment pour le marquage de rhCgA, à une activité spécifique de 400 kBq/µg. Les standards sont les mêmes que ceux décrits précédemment dans l'exemple 1.

Le tampon utilisé pour l'essai est constitué de Na₂HPO₄/KH₂PO₄ 50 mmol/l, EDTA 1 mmol/l, NaN₃ 15 mmol/l, BSA 1 %, pH 7,0.

Pour chaque dosage, on introduit 1 ml de tampon d'essai et 50 µl d'échantillon standard ou d'échantillon à doser dans les tubes revêtus de l'anticorps CGS06. On laisse incuber pendant 18 heures à la température ambiante. On lave alors les tubes deux fois avec 2 ml d'une solution de Tween 20 à 0,3 %. On ajoute alors 1 ml de l'anticorps CGS30 marqué dans chaque tube et on laisse incuber pendant 2 heures à la température ambiante sous agitation douce. On lave de nouveau les tubes comme précédemment décrit et on détermine leur radioactivité sur un compteur gamma.

Comme dans l'exemple 1, on obtient une courbe d'étalonnage à partir des échantillons standards de concentrations connues, et on détermine la concentration en chromogranine A de l'échantillon à doser en se référant à la courbe standard.

Pour vérifier la spécificité du dosage, on effectue celui-ci sur un pool normal (NP) et sur deux pools pathologiques obtenus à partir de plasmas EDTA provenant respectivement de patients atteints de phéochromocytome (PP) ou de carcinoïdes intestinaux (CP).

On évalue cette spécificité en déterminant les rapports de concentration de CgA entre le pool pathologique PP ou CP et le pool NP.

Dans le cas de cet exemple, les rapports de concentration en CgA PP/NP et CP/NP sont les suivants :
PP/NP = 9,9
CP/NP = 18, 2

Dans le cas du dosage de l'exemple 1, les rapports étaient les suivants :
PP/NP = 16,9
CP/NP = 40

A titre comparatif, le pourcentage de liaison obtenu pour l'échantillon standard à 1200 ng/ml est de 65,51.

Cette combinaison d'anticorps permet donc d'obtenir un signal élevé et de différencier les deux pools pathologiques.

Dans le tableau 3 annexé, on a donné les résultats obtenus en effectuant le même dosage sandwich avec des couples d'anticorps différents.

Ces résultats montrent que les deux anticorps CGS06 et CGS30 associés entre eux ou à l'anticorps CGS20 donnent un signal satisfaisant et permettent de discriminer les deux pools pathologiques.

A titre comparatif, on a effectué le même dosage en utilisant d'autres anticorps du tableau 2, mais ceux-ci n'ont pas donné de résultats satisfaisants.

Ainsi, lorsqu'on utilise CGS21 ou CGS10 comme second anticorps marqué, il est impossible de discriminer le pool pathologique PP du pool NP, le rapport PP/NP n'étant pas supérieur à 2, voire inférieur à 1.

D'autres combinaisons telles que CGS21/CGS06*, CGS21/CGS30* ou CGS32/CGS06* ne sont pas intéressantes car elles ne permettent pas de différencier les deux pools pathologiques entre eux, les rapports PP/NP et CP/NP étant voisins.

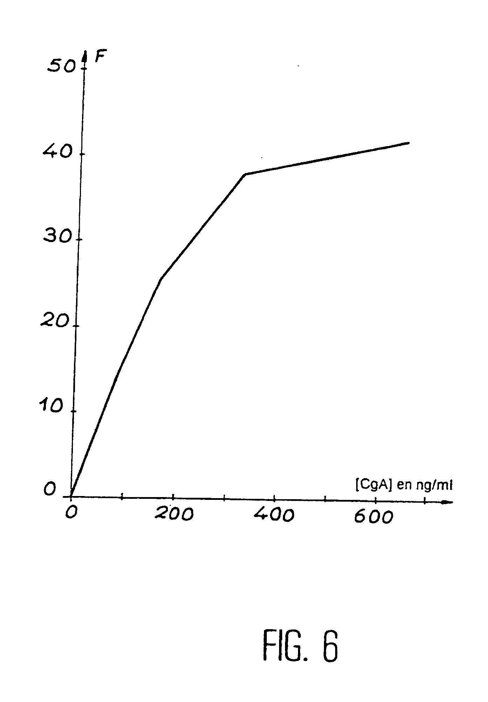

Sur la figure 5, on a illustré les résultats des dosages immunologiques des exemples 1 et 2 conformes à l'invention et de deux autres dosages utilisant la méthode sandwich comme dans l'exemple 2, mais avec la combinaison d'anticorps CGS06/CGS29* marqué et la combinaison CGS06/CGS04* marqué. Les deux anticorps CGS29 et CGS04 reconnaissent la partie C-terminale de la CgA. L'anticorps CGS29 partage le même épitope que CGS10.

Sur cette figure, les résultats sont exprimés sous la forme du rapport de concentration du plasma pathologique sur la valeur du 95ème percentile de la population normale (n = 20). Les droites indiquent la médiane pour chaque groupe.

Cette figure montre que l'on obtient des résultats équivalents avec les exemples 1 et 2. En revanche, avec le couple CGS06/CGS04* la corrélation est dégradée et un certain nombre d'échantillons ont un niveau de CgA abaissé de manière très importante. Dans le cas du dosage avec CGS06/CGS29*, le phénomène est encore amplifié, on a une diminution très importante des concentrations mesurées, le taux de CgA étant indétectable dans 50 % de la population.

Ainsi, les meilleurs résultats sont obtenus avec les anticorps de la séquence n° 2 des acides aminés 145-234 de la CgA, conformément à l'invention.

### Exemple 3 : Dosage de la chromogranine A par une méthode sandwich en phase homogène, et en fluorescence (méthode FIA).

Pour ce dosage, on utilise deux anticorps différents, le premier anticorps est l'anticorps CGS06 spécifique de la séquence n° 3 des acides aminés 219-234 et le second anticorps est l'anticorps CGS30 (ou CGS33) spécifique de la séquence n° 4 des acides aminés 145 à 197. Le premier anticorps CGS06 a été conjugué à du cryptate d'europium qui est un composé donneur d'énergie.

Le second anticorps CGS30 (ou CGS33) a été conjugué à l'allophycocyanine qui est un composé accepteur d'énergie.

Le cryptate d'europium utilisé est le cryptate Eu trisbipyridine diamine (TBP (Eu²⁺) cryptate). Il est décrit dans le document EP-A-321 353 [11] et répond à la formule :

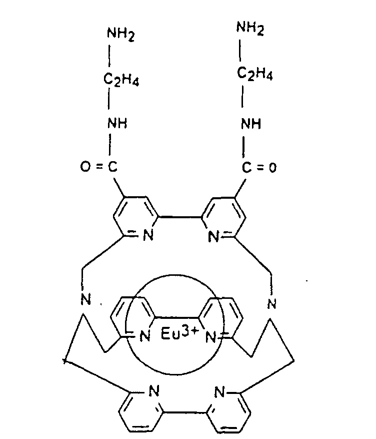

Le conjugué CGS06-cryptate est préparé par la méthode décrite par Lopez et al dans Clin. Chem., 39, 1993, pages 196-201 [12].

Le conjugué CGS30 (ou CGS33)-allophycocyanine (APC) est préparé par la méthode décrite dans WO92/01225 [13]. On utilise pour le dosage les tampons suivants :
- tampon conjugué : PO₄ 50 mM pH 7,0, BSA 0,1 %, EDTA 1,5 mM, immunoglobulines de souris normale 1,5 mM, KF 600 mM, Kathon 0,1 %,
- tampon d'incubation : PO₄ 50 mM pH 7, 0, BSA 1 %, EDTA 1,5 mM, sérum humain normal 10 %, Kathon 0,1 %.

On introduit dans chaque puits d'une microplaque noire à 96 puits, et dans cet ordre :
- 100 µl de conjugué CGS06-cryptate dilué dans le tampon conjugué, à une concentration de 0,5 µg/ml,
- 100 µl de conjugué CGS30(ou CGS33)-APC dilué dans le tampon conjugué, à une concentration de 5 µg/ml, et
- 100 µl de calibrateur ou d'échantillon à doser dilué dans le tampon d'incubation.

On laisse incuber pendant 1 heure à 37°C, puis on mesure la fluorescence de chaque puits à 620 et 665 nm sur un appareil dédié, comme il est décrit par Mathis dans Clin. Chem. 39, 1993, pages 1953-1959 [14].

Les résultats obtenus sont donnés sur la figure 6 qui représente la concentration en CgA (en ng/ml) en fonction du signal détecté.

La sensibilité du dosage ainsi réalisé avec les anticorps CGS06 et CGS30 (ou CGS33) est de 1,5 ng/ml, et les coefficients de variation intra-essai sont de 2,88% pour une concentration de 15,7 ng/ml en CgA, de 1,5% pour une concentration de 50 ng/ml et inférieurs à 1% pour toute concentration supérieure à 50 ng/ml. Les coefficients de variations inter-dosages sont de 1,2% pour une concentration en CgA de 70 ng/ml (n=11 dosages), de 1,7% pour une concentration de 409 ng/ml (n=10).

On réalise un test de récupération en surchargeant des échantillons pathologiques de sérum A à H ayant une concentration connue en CgA avec une quantité définie de CgA humaine recombinante purifiée exempte de fragments contaminants. Le taux de récupération est calculé en faisant le rapport entre la concentration en CgA humaine mesurée avec ce dosage et la concentration théorique du mélange. Les résultats de ce test sont donnés dans le tableau 4. Les taux de récupération obtenus sont compris entre 93,4% et 106%, ce qui indique que les anticorps choisis pour ce dosage permettent une identité de reconnaissance entre la CgA humaine contenue dans des échantillons pathologiques et la CgA humaine recombinante purifiée exempte de fragments contaminants.

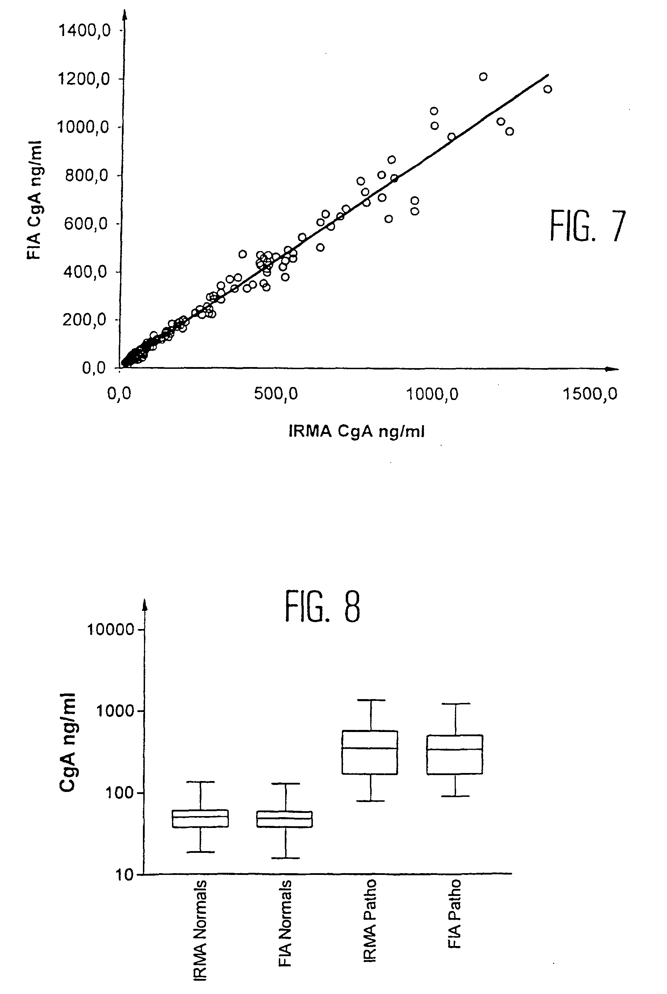

Une corrélation a été réalisée, entre le dosage IRMA décrit dans l'exemple 2 (CGS06 et CGS30) et le dosage FIA de l'exemple 3 utilisant les anticorps CGS06 et CGS33, sur 100 échantillons normaux et 95 échantillons de malades atteints d'une pathologie tumorale de type neuroendocrine. Les résultats sont reportés sur la figure 7. L'équation de la droite obtenue correspond à y = 0,8964 x = 7,4205 et le coefficient de détermination obtenu est de 0,9776.

L'étude comparative des 100 échantillons normaux et 95 échantillons pathologiques, dont les résultats sont reportés sur la figure 8 et dans le tableau 5, montre une bonne discrimination entre ces deux types d'échantillons, ainsi qu'une grande concordance entre le dosage FIA (CGS06/CGS33) et le dosage IRMA (CGS06/CGS30) de l'exemple 3 décrit dans l'exemple 2. Cette concordance confirme l'importance de l'utilisation d'un anticorps dirigé contre la séquence n° 4 (séquence 145-197) en association avec CGS06 dirigé contre la séquence n° 3 (219-234).

### Références citées

[1] : Konecki et al J. Biol. Chem., 262, 1987, pages 17026-17030.
[2] : Corti et al, Br. J. Cancer, 73, 1996, pages 924-932.
[3] : US-A-4 758 522.
[4] : Syversen et al, Acta Oncologica, 32, n°2, 1993, pages 161-165.
[5] : Kohler et Milstein, Nature, 1975, 256, pages 495-497.
[6] : O'Connor et al, New Engl. J. Med., 1984, 311, pages 764-770.
[7] : 0'Farrel, J. Biol. Chem. 72, 1975, pages 248-254
[8] : Bader et Aunis. Neuroscience, 8, 1983, pages 165-181.
[9] : Taupenot et al, Regul Pept., 56, 1995, pages 71-88.
[10] : Goumon et al, Eur J. Biochem.,235, pages 516-525.
[11] : EP-A-0 321 353.
[12] : Lopez et al Clin. Chem., 39, 1993, pages 196-201.
[13] : WO 92/01225
[14] : Mathis, Clin. Chem. 39, 1993, pages 1953-1959.
[15] : Munoz, Lab. Invest., 1991, vol. 64, pages 826-832.
[16] : Degorce et al, Tumor Biol., 18, n° suppl. 1, 1997, page 112.

**Tableau 1**

| **Anticorps** | **Isotype** | **Stoechiométrie** | **Groupe épitopique** |
|---|---|---|---|
| CGS01 | IgG1 | 0,431 | 1 |
| CGS03 | IgG1 | n.t. | |
| CGS04 | IgG2b | 0,365 | |
| CGS12 | IgG1 | 0,364 | |
| CGS06 | IgG2a | 0,503 | 2 |
| CGS08 | IgG1 | 0,457 | |
| CGS10 | IgG1 | 0,182 | 3a |
| CGS05 | IgG1 | 0,157 | |
| CGS23 | IgG2a | 0, 604 | |
| CGS24 | IgG2a | 0,521 | |
| CGS25 | IgG2a | 0,553 | |
| CGS26 | IgG2a | 0,356 | |
| CGS29 | IgG1 | 0,621 | 3b |
| CGS34 | IgG1 | 0,536 | |
| CGS35 | IgG1 | 0,539 | 3c |
| CGS30 | IgG2a | 0,327 | 4 |
| CGS31 | IgG1 | 0,198 | |
| CGS33 | IgG1 | 0,383 | |
| CGS36 | IgG1 | 0,184 | |
| CGS17 | IgG1 | 0,162 | 5 |
| CGS19 | IgG1 | 0,361 | |
| CGS20 | IgG1 | 0,254 | 6 |
| CGS21 | IgG1 | 0,183 | 7 |
| CGS32 | IgG1 | 0,262 | 8 |

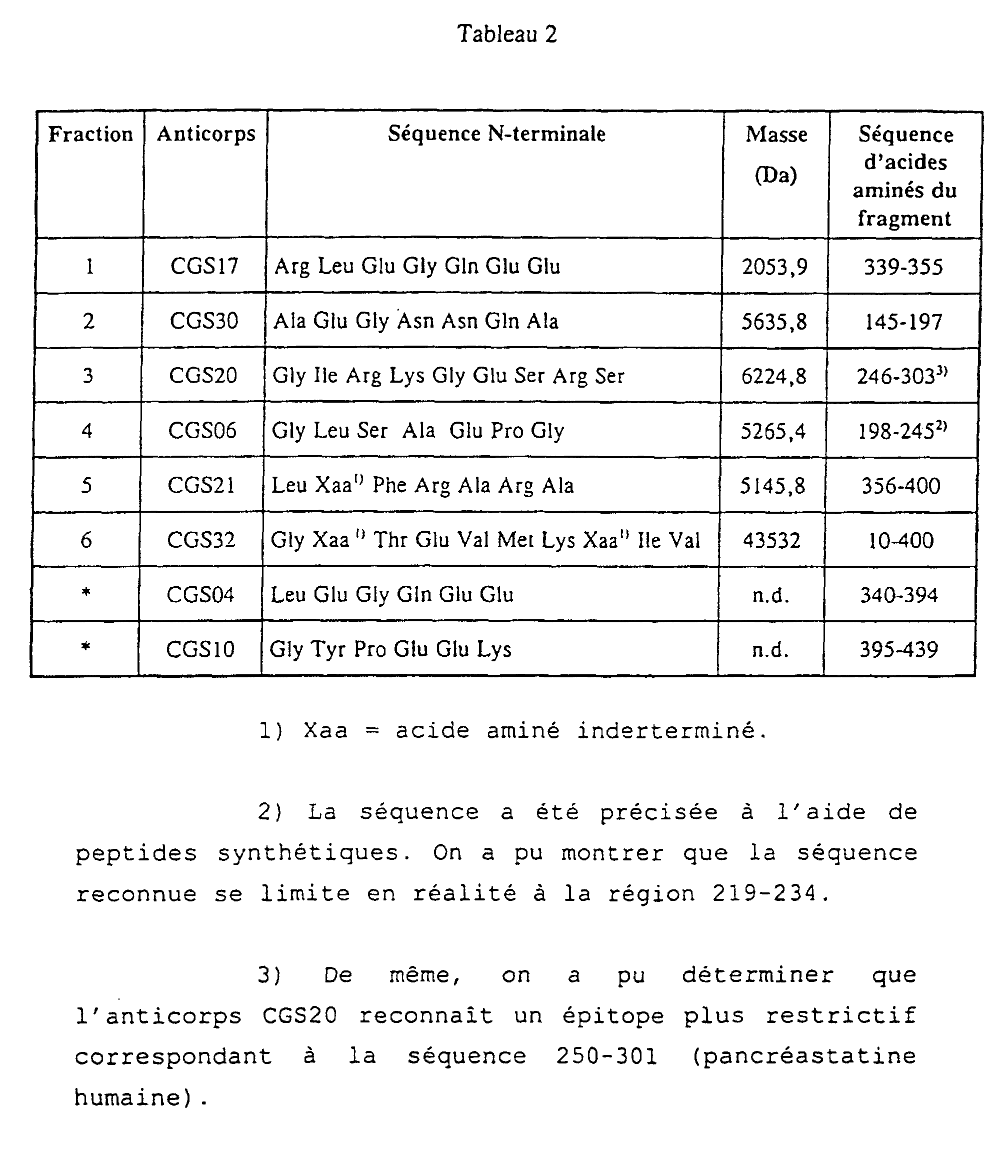

**Tableau 3**

| **Combinaison d'anticorps** | | **Pourcentage de liaison avec standard 1200 ng/ml** | **Rapport des concentrations en CgA** | |
|---|---|---|---|---|
| 1er anticorps sur phase solide | 2nd anticorps marqué à ¹²⁵I | | PP/NP | CP/NP |
| CGS06 | CGS30* | 65,51 | 9,9 | 18,2 |
| CGS30 | CGS06* | 57,56 | 16,0 | 25,0 |
| CGS06 | CGS20* | 12,8 | 4,0 | 23,2 |
| CGS20 | CGS06* | 25,43 | 2,0 | 15,6 |
| CGS30 | CGS20* | 22,04 | 5,6 | 21,8 |
| CGS20 | CGS30* | 43,22 | 2,6 | 12,8 |

**Tableau 4**

| Sérums | CgA humaine recombinante ajoutée (ng/ml) | CgA dosée (ng/ml) | Concentration théorique en CgA (ng/ml) | % de récupération |
|---|---|---|---|---|
| A | 0 | 61,3 | 61,3 | 100,0% |
| | 50 | 109,3 | 111,3 | 98,2% |
| | 200 | 250,9 | 261,3 | 96,0% |
| | 400 | 438,9 | 461,3 | 95,1% |
| B | 0 | 183,9 | 183,9 | 100,0% |
| | 50 | 243,6 | 233,9 | 104,1% |
| | 200 | 397,5 | 383,9 | 103,5% |
| | 400 | 610,5 | 583,9 | 104,6% |
| C | 0 | 118,7 | 118,7 | 100,0% |
| | 50 | 172,7 | 168,7 | 102,4% |
| | 200 | 328,4 | 318,7 | 103,0% |
| | 400 | 538 | 518,7 | 103,7% |
| D | 0 | 528 | 528 | 100,0% |
| | 50 | 584,6 | 578 | 101,1% |
| | 200 | 719,8 | 728 | 98,9% |
| | 400 | 889,3 | 928 | 95,8% |
| E | 0 | 40,1 | 40,1 | 100,0% |
| | 50 | 90,3 | 90,1 | 100,2% |
| | 200 | 228,6 | 240,1 | 95,2% |
| | 400 | 410,9 | 440,1 | 93,4% |
| F | 0 | 45,7 | 45,7 | 100,0% |
| | 50 | 100,1 | 95,7 | 104,6% |
| | 200 | 260,5 | 245,7 | 106,0% |
| | 400 | 467,3 | 445,7 | 104,8% |
| G | 0 | 187,7 | 187,7 | 100,0% |
| | 50 | 235,5 | 237,7 | 99,1% |
| | 200 | 379,9 | 387,7 | 98,0% |
| | 400 | 561 | 587,7 | 95,5% |
| H | 0 | 206,7 | 206,7 | 100,0% |
| | 50 | 263,5 | 256,7 | 102,6% |
| | 200 | 419,1 | 406,7 | 103,0% |
| | 400 | 614,9 | 606,7 | 101,4% |

**Tableau 5**

| **X Marqueurs** | **IRMA Normaux** | **FIA Normaux** | **IRMA Patho** | **FIA Patho** |
|---|---|---|---|---|
| Nombre de valeurs | 100 | 100 | 95 | 95 |
| Minimum | 19 | 16 | 79 | 90 |
| 25 % Percentile | 37 | 37 | 170 | 160 |
| Médian | 50 | 48 | 350 | 330 |
| 75 % Percentil | 61 | 59 | 560 | 500 |
| Maximum | 130 | 130 | 1400 | 1200 |

## Revendications

1. Procédé de dosage immunologique de la chromogranine A (CgA) humaine présente dans un échantillon utilisant au moins un anticorps monoclonal ou polyclonal qui se lie spécifiquement à un épitope situé dans la séquence d'acides aminés allant de l'acide aminé n° 145 à l'acide aminé n° 234 à partir de l'extrémité N-terminale de la CgA humaine.

2. Procédé selon la revendication 1, dans lequel le dosage est effectué en phase homogène ou en phase hétérogène.

3. Procédé selon la revendication 1 ou 2, dans lequel le dosage immunologique est un dosage par compétition entre la chromogranine A de l'échantillon et une quantité donnée de chromogranine A humaine marquée, pour les sites dudit anticorps.

4. Procédé selon la revendication 3, dans lequel l'anticorps est spécifique d'un épitope situé dans la séquence allant de l'acide aminé n° 219 à l'acide aminé n° 234 de la CgA humaine ou dans la séquence allant de l'acide aminé n° 145 à l'acide aminé n° 197 de la CgA humaine.

5. Procédé selon l'une quelconque des revendications 3 et 4, dans lequel la chromogranine A marquée est marquée par un marqueur du groupe comprenant les radio-éléments, les éléments luminescents, les enzymes, les chromophores fluorescents, les chromophores absorbant la lumière, et tout autre ligand permettant une mesure quantitative directe ou indirecte.

6. Procédé selon la revendication 1 ou 2, dans lequel le dosage immunologique est un dosage de type sandwich utilisant un premier anticorps monoclonal ou polyclonal qui se lie spécifiquement à un premier épitope de la CgA humaine, et un second anticorps monoclonal ou polyclonal qui se lie spécifiquement à un second épitope de la CgA humaine différent du premier, l'un au moins des épitopes étant situé dans la séquence allant de l'acide aminé n° 145 à l'acide aminé n° 234 de la CgA humaine et l'un des deux anticorps étant marqué.

7. Procédé selon la revendication 6, dans lequel le premier et le second anticorps sont choisis parmi les anticorps spécifiques d'épitopes situés dans les séquences allant de l'acide aminé n° 145 à l'acide aminé n° 197 et/ou allant de l'acide aminé n° 219 à l'acide aminé n° 234 de la CgA humaine.

8. Procédé selon la revendication 6, dans lequel le premier anticorps est spécifique d'un épitope situé dans la séquence allant de l'acide aminé n° 145 à l'acide aminé n° 197 de la CgA humaine, ou dans la séquence allant de l'acide aminé n° 219 à l'acide aminé n° 234 de la CgA humaine, et le second anticorps est spécifique d'un épitope situé dans la séquence allant de l'acide aminé n° 250 à l'acide aminé n° 301 de la CgA humaine.

9. Procédé selon l'une quelconque des revendications 6 à 8, dans lequel l'anticorps marqué est marqué par un marqueur du groupe comprenant les radio-éléments, les éléments luminescents, les enzymes, les chromophores fluorescents, les chromophores absorbant la lumière, et tout autre ligand permettant une mesure quantitative directe ou indirecte.

10. Procédé selon l'une quelconque des revendications 6 à 8, dans lequel l'anticorps marqué est marqué par un composé luminescent donneur d'énergie et l'autre anticorps est couplé à un composé luminescent accepteur d'énergie.

11. Procédé selon la revendication 10, dans lequel le composé luminescent donneur d'énergie est un cryptate d'europium et le composé luminescent accepteur d'énergie est l'allophycocyanine.

12. Anticorps monoclonal capable de se lier spécifiquement à un épitope situé dans la séquence allant de l'acide aminé n° 219 à l'acide aminé n° 234 à partir de l'extrémité N-terminale de la chromogranine A humaine.

13. Anticorps monoclonal capable de se lier spécifiquement à un épitope situé dans la séquence allant de l'acide aminé n° 145 à l'acide aminé n° 197 à partir de l'extrémité N-terminale de la chromogranine A humaine.

14. Réactif immunologique comprenant l'anticorps monoclonal de la revendication 12 ou 13 couplé à un marqueur détectable choisi parmi les radio-éléments, les composés luminescents donneurs d'énergie, les composés luminescents accepteurs d'énergie, et tout autre ligand permettant une mesure quantitative directe ou indirecte.

15. Trousse de dosage immunologique de la chromogranine A (CgA) humaine comprenant
- un premier anticorps, et
- un second anticorps,
l'un des deux anticorps au moins étant un anticorps spécifique d'un épitope situé dans la séquence allant de l'acide aminé n° 145 à l'acide aminé n° 234 à partir de l'extrémité N-terminale de la CgA humaine.

16. Trousse selon la revendication 15, dans laquelle le premier anticorps et le second anticorps sont choisis parmi les anticorps spécifiques d'épitopes situés dans les séquences allant de l'acide aminé n° 145 à l'acide aminé n° 197 et/ou allant de l'acide aminé n° 219 à l'acide aminé n° 234 de la CgA humaine.

17. Trousse selon la revendication 15, dans laquelle l'un des anticorps est spécifique d'un épitope situé dans la séquence allant de l'acide aminé n° 145 à l'acide aminé n° 197 ou dans la séquence allant de l'acide aminé n° 219 à l'acide aminé n° 234, et l'autre anticorps est spécifique d'un épitope situé dans la séquence allant de l'acide aminé n° 250 à l'acide aminé n° 301 de la CgA humaine.

18. Trousse selon l'une quelconque des revendications 15 à 17, dans laquelle l'un des anticorps est couplé à un composé luminescent donneur d'énergie et l'autre anticorps est couplé à un composé luminescent accepteur d'énergie.

19. Trousse selon la revendication 18, dans laquelle le composé luminescent donneur d'énergie est un cryptate d'europium et le composé luminescent accepteur d'énergie est l'allophycocyanine.

20. Trousse de dosage immunologique par compétition comprenant un anticorps spécifique d'un épitope situé dans la séquence allant de l'acide aminé n° 145 à l'acide aminé n° 234 de la CgA.

21. Trousse de dosage selon la revendication 20, dans laquelle l'anticorps est spécifique d'un épitope situé dans la séquence allant de l'acide aminé n° 219 à l'acide aminé n° 234 ou dans la séquence allant de l'acide aminé n° 145 à l'acide aminé n° 197 de la CgA humaine.

## Patentansprüche

1. Verfahren zum immunologischen Nachweis von menschlichem Chromogranin A (CgA), das in einer Probe vorliegt, unter Verwendung mindestens eines monoklonalen oder polyklonalen Antikörpers, der sich spezifisch an ein Epitop bindet, das sich in der Aminosäuresequenz von Aminosäure Nr. 145 bis Aminosäure Nr. 234, ausgehend von N-Terminus des menschlichen CgA, befindet.

2. Verfahren nach Anspruch 1, wobei der Nachweis in homogener oder heterogener Phase erfolgt.

3. Verfahren nach Anspruch 1 oder 2, wobei der immunologische Nachweis ein Nachweis durch Konkurrenz zwischen dem Chromogranin A der Probe und einer gegebenen Menge an markiertem menschlichem Chromogranin A um die Stellen des Antikörpers ist.

4. Verfahren nach Anspruch 3, wobei der Antikörper für ein Epitop spezifisch ist, das sich in der Sequenz von Aminosäure Nr. 219 bis Aminosäure Nr. 234 des menschlichen CgA oder in der Sequenz von Aminosäure Nr. 145 bis Aminosäure Nr. 197 des menschlichen CgA befindet.

5. Verfahren nach einem der Ansprüche 3 und 4, wobei das markierte Chromogranin A durch einen Marker aus der Gruppe markiert ist, die radioaktive Elemente, lumineszente Elemente, Enzyme, fluoreszierende Chromophore, das Licht absorbierende Chromophore und jeden anderen Liganden umfasst, der eine direkte oder indirekte quantitative Messung ermöglicht.

6. Verfahren nach Anspruch 1 oder 2, wobei der immunologische Nachweis ein Nachweis des Sandwich-Typs ist, wobei ein erster monoklonaler oder polyklonaler Antikörper, der sich spezifisch an ein erstes Epitop des menschlichen CgA bindet, und ein zweiter monoklonaler oder polyklonaler Antikörper verwendet werden, der sich spezifisch an ein zweites, von dem ersten verschiedenes Epitop des menschlichen CgA bindet, wobei sich mindestens eines der Epitope in der Sequenz von Aminosäure Nr. 145 bis Aminosäure Nr. 234 des menschlichen CgA befindet und einer der beiden Antikörper markiert ist.

7. Verfahren nach Anspruch 6, wobei der erste und der zweite Antikörper unter den Antikörpern ausgewählt sind, die für Epitope spezifisch sind, die sich in den Sequenzen von Aminosäure Nr. 145 bis Aminosäure Nr. 197 und/oder von Aminosäure Nr. 219 bis Aminosäure Nr. 234 des menschlichen CgA befinden.

8. Verfahren nach Anspruch 6, wobei der erste Antikörper für ein Epitop spezifisch ist, das sich in der Sequenz von Aminosäure Nr. 145 bis Aminosäure Nr. 197 des menschlichen CgA oder in der Sequenz von Aminosäure Nr. 219 bis Aminosäure Nr. 234 des menschlichen CgA befindet, und der zweite Antikörper für ein Epitop spezifisch ist, das sich in der Sequenz von Aminosäure Nr. 250 bis Aminosäure Nr. 301 des menschlichen CgA befindet.

9. Verfahren nach einem der Ansprüche 6 bis 8, wobei der markierte Antikörper durch einen Marker aus der Gruppe markiert ist, die radioaktive Elemente, lumineszente Elemente, Enzyme, fluoreszierende Chromophore, das Licht absorbierende Chromophore und jeden anderen Liganden umfasst, der eine direkte oder indirekte quantitative Messung ermöglicht.

10. Verfahren nach einem der Ansprüche 6 bis 8, wobei der markierte Antikörper durch eine Energie abgebende lumineszente Verbindung markiert ist und der andere Antikörper mit einer Energie aufnehmenden lumineszenten Verbindung gekuppelt ist.

11. Verfahren nach Anspruch 10, wobei die Energie abgebende lumineszente Verbindung ein Europiumkryptat ist und die Energie aufnehmende lumineszente Verbindung Allophycocyanin ist.

12. Monoklonaler Antikörper, der sich spezifisch an ein Epitop binden kann, das sich in der Sequenz von Aminosäure Nr. 219 bis Aminosäure Nr. 234, ausgehend von N-Terminus des menschlichen Chromogranins A, befindet.

13. Monoklonaler Antikörper, der sich spezifisch an ein Epitop binden kann, das sich in der Sequenz von Aminosäure Nr. 145 bis Aminosäure Nr. 197, ausgehend von N-Terminus des menschlichen Chromogranins A, befindet.

14. Immunologisches Reagenz, umfassend den monoklonalen Antikörper des Anspruchs 12 oder 13, gekuppelt an einen nachweisbaren Marker, der aus radioaktiven Elementen, Energie abgebenden lumineszenten Verbindungen, Energie aufnehmenden lumineszenten Verbindungen und jedem anderen Liganden ausgewählt ist, der eine direkte oder indirekte quantitative Messung ermöglicht.

15. Kit zum immunologischen Nachweis von menschlichem Chromogranin A (CgA), das folgendes umfasst
- einen ersten Antikörper und
- einen zweiten Antikörper,
wobei mindestens einer der beiden Antikörper ein Antikörper ist, der für ein Epitop spezifisch ist, das sich in der Sequenz von Aminosäure Nr. 145 bis Aminosäure Nr. 234, ausgehend von N-Terminus des menschlichen CgA, befindet.

16. Kit nach Anspruch 15, wobei der erste Antikörper und der zweite Antikörper aus den Antikörpern ausgewählt sind, die für Epitope spezifisch sind, die sich in den Sequenzen von Aminosäure Nr. 145 bis Aminosäure Nr. 197 und/oder von Aminosäure Nr. 219 bis Aminosäure Nr. 234 des menschlichen CgA befinden.

17. Kit nach Anspruch 15, wobei einer der Antikörper für ein Epitop spezifisch ist, das sich in der Sequenz von Aminosäure Nr. 145 bis Aminosäure Nr. 197 oder in der Sequenz von Aminosäure Nr. 219 bis Aminosäure Nr. 234 befindet, und der andere Antikörper für ein Epitop spezifisch ist, das sich in der Sequenz von Aminosäure Nr. 250 bis Aminosäure Nr. 301 des menschlichen CgA befindet.

18. Kit nach einem der Ansprüche 15 bis 17, wobei einer der Antikörper an eine Energie abgebende lumineszente Verbindung gekuppelt ist und der andere Antikörper an eine Energie aufnehmende lumineszente Verbindung gekuppelt ist.

19. Kit nach Anspruch 18, wobei die Energie abgebende lumineszente Verbindung ein Europiumkryptat ist und die Energie aufnehmende lumineszente Verbindung Allophycocyanin ist.

20. Kit zum immunologischen Nachweis durch Konkurrenz, das einen Antikörper umfasst, der für ein Epitop spezifisch ist, das sich in der Sequenz von Aminosäure Nr. 145 bis Aminosäure Nr. 234 des CgA befindet.

21. Kit zum immunologischen Nachweis nach Anspruch 20, wobei der Antikörper für ein Epitop spezifisch ist, das sich in der Sequenz von Aminosäure Nr. 219 bis Aminosäure Nr. 234 oder in der Sequenz von Aminosäure Nr. 145 bis Aminosäure Nr. 197 des menschlichen CgA befindet.

## Claims

1. Immunoassay method for human chromogranin A (CgA) present in a sample using at least one monoclonal or polyclonal antibody which binds specifically to an epitope positioned in the sequence of amino acids extending from amino acid No. 145 to amino acid No. 234 from the N-terminal end of human CgA.

2. Method according to Claim 1, in which the assay is conducted in homogeneous phase or in heterogeneous phase.

3. Method according to Claim 1 or 2, in which the immunoassay is a competitive assay between the chromogranin A of the sample and a given quantity of labelled human chromogranin A, for the sites of said antibody.

4. Method according to Claim 3, in which the antibody is specific for an epitope positioned in the sequence extending from amino acid No. 219 to amino acid No. 234 of human CgA, or in the sequence extending from amino acid No. 145 to amino acid No. 197 of human CgA.

5. Method according to either one of Claims 3 and 4, in which the labelled chromogranin A is labelled with a label from the group comprising radioelements, luminescent elements, enzymes, fluorescent chromophores, light-absorbing chromophores, and any other ligand allowing direct or indirect quantitative measurement.

6. Method according to Claim 1 or 2, in which the immunoassay is a sandwich-type assay using a first monoclonal or polyclonal antibody which binds specifically to a first epitope of human CgA, and a second monoclonal or polyclonal antibody which binds specifically to a second epitope of human CgA different from the first, at least one the epitopes being positioned in the sequence extending from amino acid No. 145 to amino acid No. 234 of human CgA and one of the two antibodies being labelled.

7. Method according to Claim 6, in which the first and the second antibodies are chosen from the antibodies specific for epitopes positioned in the sequences extending from amino acid No. 145 to amino acid No. 197 and/or extending from amino acid No. 219 to amino acid No. 234 of human CgA.

8. Method according to Claim 6, in which the first antibody is specific for an epitope positioned in the sequence extending from amino acid No. 145 to amino acid No. 197 of human CgA, or in the sequence extending from amino acid No. 219 to amino acid No. 234 of human CgA, and the second antibody is specific to an epitope positioned in the sequence extending from amino acid No. 250 to amino acid No. 301 of human CgA.

9. Method according to any one of Claims 6 to 8, in which the labelled antibody is labelled with a label from the group comprising radioelements, luminescent elements, enzymes, fluorescent chromophores, light-absorbing chromophores, and any other ligand allowing direct or indirect quantitative measurement.

10. Method according to any one of Claims 6 to 8, in which the labelled antibody is labelled with an energy-donor luminescent compound, and the other antibody is coupled to an energy-acceptor luminescent compound.

11. Method according to Claim 10, in which the energy-donor luminescent compound is a europium cryptate and the energy-acceptor luminescent compound is allophycocyanin.

12. Monoclonal antibody capable of binding specifically to an epitope positioned in the sequence extending from amino acid No. 219 to amino acid No. 234 from the N-terminal end of human chromogranin A.

13. Monoclonal antibody capable of binding specifically to an epitope positioned in the sequence extending from amino acid No. 145 to amino acid No.197 from the N-terminal end of human chromogranin A.

14. Immunological reagent containing the monoclonal antibody of Claim 12 or 13 coupled to a detectable label chosen from radioelements, energy-donor luminescent compounds, energy-acceptor luminescent compounds, and any other ligand allowing direct or indirect quantitative measurement.

15. Immunoassay kit for human chromogranin A (CgA) comprising:
- a first antibody, and
- a second antibody,
at least one of the two antibodies being an antibody specific for an epitope positioned in the sequence extending from amino acid No. 145 to amino acid No. 234 from the N-terminal end of human CgA.

16. Kit according to Claim 15, in which the first antibody and the second antibody are chosen from the antibodies specific for epitopes positioned in the sequences extending from amino acid No. 145 to amino acid No. 197 and/or extending from amino acid No. 219 to amino acid No. 234 of human CgA.

17. Kit according to Claim 15, in which one of the antibodies is specific for an epitope positioned in the sequence extending from amino acid No. 145 to amino acid No. 197 or in the sequence extending from amino acid No. 219 to amino acid No. 234, and the other antibody is specific for an epitope positioned in the sequence extending from amino acid No. 250 to amino acid No. 301 of human CgA.

18. Kit according to any one of Claims 15 to 17, in which one of the antibodies is coupled to an energy-donor luminescent compound and the other antibody is coupled to an energy-acceptor luminescent compound.

19. Kit according to Claim 18, in which the energy-donor luminescent compound is a europium cryptate and the energy-acceptor luminescent compound is allophycocyanin.

20. Competitive immunoassay kit comprising an antibody specific for an epitope positioned in the sequence extending from amino acid No. 145 to amino acid No. 234 of CgA.

21. Assay kit according to Claim 20, in which the antibody is specific for an epitope positioned in the sequence extending from amino acid No. 219 to amino acid No. 234 or in the sequence extending from amino acid No. 145 to amino acid No. 197 of human CgA.
